(19) Europäisches Patentamt / European Patent Office / Office européen des brevets

(11) **EP 4 331 485 A1**

(12) **DEMANDE DE BREVET EUROPEEN**

(43) Date de publication:
**06.03.2024 Bulletin 2024/10**

(21) Numéro de dépôt: **23193609.7**

(22) Date de dépôt: **25.08.2023**

(51) Classification Internationale des Brevets (IPC):
**A61B 5/145** (2006.01)  **G01N 33/49** (2006.01)
*A61B 5/083* (2006.01)  *A61B 5/1455* (2006.01)
*A61B 5/1477* (2006.01)  *G01N 33/00* (2006.01)
*G01N 33/02* (2006.01)  *G01N 33/18* (2006.01)
*G01N 33/24* (2006.01)  *G01N 33/483* (2006.01)

(52) Classification Coopérative des Brevets (CPC):
**A61B 5/14542; G01N 33/4925;** A61B 5/0836;
A61B 5/14551; A61B 5/1477; G01N 33/0047;
G01N 33/025; G01N 33/18; G01N 33/4833;
G01N 2033/243

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(30) Priorité: **28.08.2022 FR 2208591**

(71) Demandeurs:
• **Commissariat à l'énergie atomique
et aux énergies alternatives
75015 Paris (FR)**

• **L3 Medical
38070 Saint Quentin Fallavier (FR)**

(72) Inventeurs:
• **GRANGEAT, Pierre**
**38054 Grenoble cedex 09 (FR)**
• **JAILLET-CASILLAS, Myrna Violeta**
**38054 Grenoble cedex 09 (FR)**
• **STOCARD, Fabien**
**94370 SUCY EN BRIE (FR)**

(74) Mandataire: **INNOV-GROUP**
**310, avenue Berthelot**
**69372 Lyon Cedex 08 (FR)**

(54) **DISPOSITIF PORTABLE D'ESTIMATION D'UNE CONCENTRATION DE GAZ DÉGAGÉ PAR UN MILIEU**

(57) Dispositif de mesure (1), destiné à être disposé contre un milieu, le dispositif s'étendant entre une face de contact (10), destinée à être appliquée contre le milieu et une extrémité distale (4), le dispositif comportant une paroi latérale (5), s'étendant entre la face de contact et l'extrémité distale, le dispositif comportant :
- au niveau de la face de contact (10), au moins une ouverture d'admission (12), configurée pour collecter un gaz d'intérêt transcutané, émis à travers le milieu, l'ouverture d'admission étant pratiquée à travers la face de contact;
- une chambre de mesure (20), comportant un capteur de gaz (23), le capteur de gaz étant configuré pour mesurer une concentration de gaz d'intérêt s'écoulant à travers la chambre de mesure;
- une chambre de collecte (30), reliée à la chambre de mesure, et délimitée par une ouverture sur la paroi latérale, la chambre de collecte comportant au moins une ouverture latérale (34), ménagée à travers la face latérale, ou sur la paroi supérieure de la chambre de collecte de façon à admettre un gaz porteur dans la chambre de collecte ;
le dispositif étant caractérisé en ce que :

- la chambre de mesure (20) est disposée entre la face de contact (10) et la chambre de collecte (30) ;
- le dispositif comporte une pompe (41), configurée pour entraîner un gaz porteur à travers la chambre de collecte vers une ouverture d'évacuation (42), l'entraînement du gaz porteur induisant une aspiration du gaz d'intérêt de la face de contact vers la chambre de collecte, à travers la chambre de mesure

EP 4 331 485 A1

**Fig. 1A**

**Description**

**DOMAINE TECHNIQUE**

Le domaine technique de l'invention est la mesure d'un gaz dégagé par un milieu à l'aide d'un dispositif compact. Le dispositif compact est appliqué contre le milieu. Le milieu peut être la peau d'un être vivant. Le dispositif permet alors d'estimer des paramètres physiologiques, en particulier la teneur de dioxyde de carbone dissous dans le sang. Le gaz peut notamment être du dioxyde de carbone, pour des applications de capnométrie. Le milieu peut être un milieu liquide, par exemple de l'eau, ou du lisier. Le gaz peut être du dioxyde de carbone ou du méthane. Le milieu peut également être un milieu végétal, auquel cas le dispositif peut être utilisé pour étudier la respiration du milieu.

**ART ANTERIEUR**

Certaines maladies respiratoires affectent les échanges gazeux entre le sang et l'air expiré. Le sang contient des gaz dissous, dont l'oxygène et le dioxyde de carbone, dont les pressions partielles respectives reflètent les échanges gazeux se produisant au niveau des poumons et des organes.

Pour évaluer la quantité de $CO_2$ dissous dans le sang, on peut avoir recours à un prélèvement sanguin. Il s'agit d'une méthode invasive, pouvant être douloureuse et délicate à appliquer, en particulier en néonatologie. De plus, elle ne peut être appliquée que de façon ponctuelle. En dépit de ces inconvénients, sa fiabilité est validée par le corps médical et elle constitue une méthode de référence. Une autre méthode consiste à estimer la teneur du sang en $CO_2$ de façon non invasive, en effectuant une mesure de la pression partielle de $CO_2$ diffusant à travers les tissus, et notamment la peau. Cette méthode est désignée par le terme de capnométrie transcutanée.

Dans le sang, le gaz carbonique est dissous suivant une espèce moléculaire ($CO_2$), et suivant deux espèces ioniques : les ions carbonates $CO_3^{2-}$ et bicarbonates $HCO_3^-$. L'équilibre entre ces espèces dépend du pH du sang. Les ions carbonates et bicarbonates sont en équilibre avec les ions hydrogène. Ainsi, la concentration des ions carbonates ou bicarbonates influence le pH du sang. Une augmentation de la concentration de $CO_2$ dissous (hypercapnie), se traduit par une augmentation de la quantité d'ions carbonates et bicarbonates, et, par équilibre, des ions hydrogène, ce qui engendre une diminution du pH du sang, ou acidose. Une augmentation de la concentration de $CO_2$ peut survenir lorsque l'élimination du $CO_2$ par les voies respiratoires est insuffisante, par exemple dans le cas de maladies pulmonaires obstructives chroniques, (COPD) ou de maladies infectieuses affectant les poumons, un exemple étant une infection due au COVID 19.

Inversement, une diminution de la concentration de $CO_2$ dissous (hypocapnie) abaisse la concentration d'ions hydrogène, ce qui entraîne une augmentation du pH, ou alcalose. Une hypocapnie peut être causée par exemple par une hyperventilation associée à une augmentation de la fréquence respiratoire. La survenue d'une acidose ou d'une alcalose peut avoir des conséquences sur le métabolisme. Ainsi, la concentration de $CO_2$ dans le sang est un paramètres vital important, qu'il convient de surveiller régulièrement pour certains patients.

Le suivi de la concentration de $CO_2$ peut également concerner des patients en réanimation, ou chez des nouveaux-nés placés en incubateur. Cela peut également trouver des applications dans le suivi d'efforts, l'activité physique favorisant la production de gaz carbonique.

Ce type d'analyse transcutanée a été introduit dans les années 1980. L'analyse transcutanée permet d'effectuer un suivi continu, par exemple pour suivre les effets immédiats d'une prise en charge thérapeutique. Elle peut également permettre de déterminer les instants auxquels une quantification précise, par prélèvement sanguin, est nécessaire. On comprend ainsi que les méthodes invasives et non invasives peuvent être combinées : l'une est précise et ponctuelle, tandis que l'autre peut être mise en oeuvre en oeuvre de façon continue.

Le document WO2017/023500 décrit un dispositif permettant une mesure d'un gaz, en l'occurrence NO, émis à travers la peau. Le dispositif comporte une chambre de collecte, présentant une ouverture latérale. Le dispositif comporte une chambre de mesure disposée en aval de la chambre de collecte. Il en résulte que le gaz circulant dans la chambre de mesure comporte une part non négligeable d'air externe, ce qui nuit à la sensibilité.

Le document JP2010148692 décrit un dispositif permettant une mesure d'un gaz, en l'occurrence $H_2$, émis à travers la peau. Le dispositif comporte une ouverture latérale permettant soit l'admission d'un gaz dans une chambre de collecte, soit l'évacuation du gaz.

Un dispositif compact permettant la mesure du $CO_2$ transcutané a déjà été décrit dans WO2020/249466. Il s'agit d'un dispositif de mesure non invasif, porté par un utilisateur, pour estimer une concentration d'un gaz d'intérêt émis de façon transcutanée, le gaz d'intérêt pouvant par exemple être le dioxyde de carbone. La circulation du gaz dans le dispositif permet une collecte du gaz d'intérêt. Le gaz d'intérêt transcutané se propage à travers le dispositif par convection, sous l'effet d'une source de chaleur. Le dispositif comporte une face de contact, destinée à être appliquée contre l'utilisateur. Le dispositif comporte également une chambre de mesure, comportant un capteur de $CO_2$. Pour que l'augmentation de température se traduise par une convection, il est préférable que la chambre de mesure soit disposée au-dessus de la face de contact.

Les inventeurs proposent un perfectionnement du dispositif décrit dans WO2020/249466, visant à améliorer certaines performances, en particulier les performances de réponse temporelle.

## EXPOSE DE L'INVENTION

Un premier objet de l'invention est un dispositif de mesure, destiné à être disposé contre un milieu, le dispositif s'étendant entre une face de contact, destinée à être appliquée en regard du milieu et une extrémité distale, le dispositif comportant une paroi latérale, s'étendant entre la face de contact et l'extrémité distale, le dispositif comportant :

- au niveau de la face de contact, au moins une ouverture d'admission, configurée pour collecter un gaz d'intérêt émis à travers le milieu, l'ouverture d'admission étant pratiquée à travers la face de contact;
- une chambre de mesure, comportant un capteur de gaz, le capteur de gaz étant configuré pour mesurer une concentration du gaz d'intérêt s'écoulant à travers la chambre de mesure;
- une chambre de collecte, reliée à la chambre de mesure, et délimitée par une ouverture sur la paroi latérale, la chambre de collecte comportant au moins une ouverture latérale, ménagée à travers la face latérale, ou sur la paroi supérieure de la chambre de collecte de façon à admettre un gaz porteur - dans la chambre de collecte ;

le dispositif étant caractérisé en ce que :

- la chambre de mesure est disposée entre la face de contact et la chambre de collecte ;
- le dispositif comporte un moyen d'entraînement, configuré pour entraîner le gaz porteur à travers la chambre de collecte vers une ouverture d'évacuation, l'entraînement du gaz porteur induisant un transport du gaz d'intérêt de la face de contact vers la chambre de collecte, à travers la chambre de mesure.

Le moyen d'entraînement peut être un propulseur d'air ou un aspirateur d'air. Le transport du gaz d'intérêt de la face de contact vers la chambre de collecte, à travers la chambre de mesure, est effectué par diffusion. La diffusion est favorisée par une dépression formée, par le moyen d'entraînement, dans la chambre de mesure.

Le dispositif peut comporter une source de chaleur, configurée pour porter la face de contact à une température supérieure à 37°C.

Selon un mode de réalisation, l'ouverture d'évacuation étant orientée autour d'un axe d'évacuation perpendiculaire ou perpendiculaire à 30° près, à la face de contact, la chambre de collecte débouchant sur l'ouverture d'évacuation.

Selon un mode de réalisation, l'ouverture d'évacuation est disposée à travers la paroi latérale de la chambre de collecte.

Selon une possibilité, le capteur de gaz de la chambre de mesure est disposé à une distance inférieure à 8 mm de la face de contact.

Le capteur de gaz peut être un capteur optique, comportant une source de lumière infra-rouge, configurée pour émettre une lumière infra-rouge, et un photodétecteur, la chambre de mesure étant agencée de telle façon que le gaz d'intérêt s'écoule, dans la chambre de mesure, entre la source de lumière infra-rouge et le photodétecteur, selon une direction d'écoulement perpendiculaire, ou perpendiculaire à $\pm$ 30° près, à la face de contact. La source de lumière infra-rouge peut être configurée pour produire une nappe de lumière parallèle, ou parallèle à $\pm$ 30° près à la face de contact. L'épaisseur de la nappe de lumière, selon un axe transversal perpendiculaire à la face de contact, peut être inférieure à 5 mm.

La chambre de mesure peut comporter deux parois, parallèles à la face de contact, chaque paroi étant réfléchissante à l'égard de la lumière infra-rouge émise par la source infra-rouge, les parois délimitant une nappe de lumière dans laquelle se propage la lumière infra-rouge entre la source infra-rouge et le photodétecteur. Une paroi réfléchissante peut former la face de contact.

La paroi réfléchissante la plus proche de la face de contact peut être configurée pour être chauffée, de façon à chauffer le milieu.

Le dispositif peut être tel que :

- chaque paroi réfléchissante comporte des cloisons transversales ;
- chaque cloison transversale d'une paroi réfléchissante s'étend vers une paroi réfléchissante opposée,
- un espace est ménagé entre chaque cloison transversale et la paroi réfléchissante qui lui est opposée ;
- une cloison transversale d'une paroi réfléchissante s'étend entre deux cloisons transversales de la paroi réfléchissante opposée ;
- de façon que la lumière, émise par la source de lumière, se propage entre les cloisons transversales successives avant d'atteindre le détecteur.

Le débit d'air de la pompe peut s'étendre entre 0.1 et 10 mL/min.

Un autre objet de l'invention est un procédé d'estimation d'une teneur en gaz d'intérêt dans un milieu, à l'aide d'un dispositif selon le premier objet de l'invention, le dispositif étant appliqué de telle sorte que la face de contact est disposée en regard du milieu, le moyen d'entraînement du gaz porteur étant activé, le procédé comportant:

a) une estimation d'une concentration de gaz d'intérêt dans la chambre de mesure;
b) à partir de la concentration du gaz d'intérêt dans la chambre de mesure, résultant de l'étape a), estimation d'une pression partielle du gaz d'intérêt dégagé le milieu.

Le milieu peut être la peau d'un utilisateur, la peau s'étendant en dessus d'un vaisseau sanguin, L'étape b) comporte alors :

(i) à partir concentration de gaz d'intérêt dans la chambre de mesure, résultant de l'étape a), estimation d'une concentration de gaz d'intérêt transcutané ;
(ii) à partir de la concentration de gaz d'intérêt transcutané résultant de la sous-étape (i), estimation d'une concentration ou pression partielle de gaz d'intérêt dissous dans le milieu.

Le milieu à analyser peut être un milieu solide ou liquide. Le gaz porteur peut être de l'air L'invention sera mieux comprise à la lecture de l'exposé des exemples de réalisation présentés, dans la suite de la description, en lien avec les figures listées ci-dessous.

## FIGURES

La figure 1A montre un premier mode de réalisation d'un dispositif selon l'invention.
La figure 1B schématise les écoulements à l'intérieur du dispositif.
La figure 2A schématise les principaux composants d'un capteur de gaz, disposé dans la chambre de mesure du dispositif. La vue est prise dans un plan perpendiculaire à un axe transversal.
La figure 2B schématise les principaux composants d'un capteur de gaz, basé sur une absorption d'une lumière infra-rouge, et disposé dans la chambre de mesure du dispositif. La vue est prise dans un plan parallèle à l'axe transversal passant par la source de lumière infrarouge 24 et le détecteur de lumière infrarouge 25.
Les figures 3A, 3B, 3C et 3D montrent différentes configurations d'un capteur de gaz.
Les figures 4A, 4B et 4C montrent différentes configurations d'un capteur de gaz comportant plusieurs sources de lumière infra-rouge.
Les figures 5A, 5B et 5C schématisent respectivement une configuration selon l'art antérieur, selon un premier mode de réalisation de l'invention et selon un deuxième mode de réalisation de l'invention.
La figure 5D illustre une détermination d'un temps de réponse.
La figure 5E illustre une impulsion simulée de concentration de $CO_2$ dans le sang ainsi qu'une impulsion de la concentration de $CO_2$ mesurée par le capteur de gaz du dispositif.
La figure 5F illustre une détermination d'un temps de retard à partir d'une intercorrélation temporelle des courbes représentées sur la figure 5E.
La figure 6A schématise un deuxième mode de réalisation.
La figure 6B schématise une deuxième configuration modélisée, correspondant au deuxième mode de réalisation.

## EXPOSE DE MODES DE REALISATION PARTICULIERS

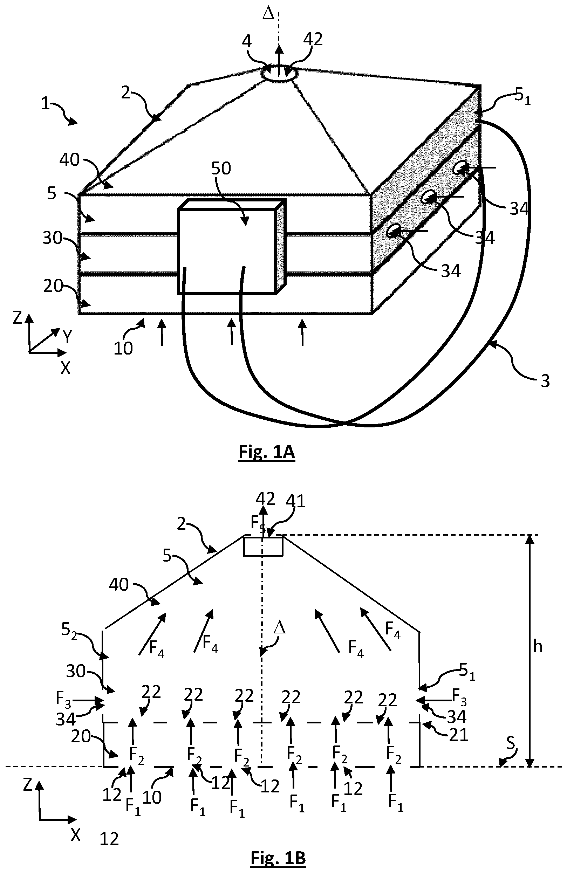

Les figures 1A et 1B sont des vues générales d'un exemple de dispositif 1 selon l'invention. Le dispositif 1 est destiné à être disposé au contact d'un milieu que l'on souhaite analyser. Dans l'exemple décrit, le milieu est de la peau S d'un utilisateur, humain ou animal. Le dispositif comporte un corps principal 2 ainsi qu'un élément de fixation 3, ce dernier étant, dans cet exemple, un bracelet. De façon alternative, le corps principal peut être disposé au contact du lobe d'une oreille, ou d'un doigt. Le support peut être une pince ou être intégré dans un casque auditif. De façon plus générale, le support est configuré pour maintenir le corps principal au contact du milieu à analyser.

Le dispositif est destiné à estimer une concentration d'un gaz d'intérêt émanant de la peau d'un utilisateur. Par gaz d'intérêt, il est entendu un gaz dont on souhaite déterminer une concentration dans un corps humain ou animal vivant, et plus particulièrement dans le sang. Dans l'exemple décrit ci-dessous, de façon non limitative, le gaz d'intérêt est le dioxyde de carbone, dont on cherche à estimer une teneur dans le sang de l'utilisateur. Selon d'autres possibilités, le gaz d'intérêt peut être, de façon non limitative, l'oxygène, l'alcool éthylique, le monoxyde de carbone, le méthane, le monoxyde d'azote, l'acétone ou l'isoprène, l'hydrogène, certaines drogues ou substances volatiles.

Le corps principal 2 comporte une face de contact 10, destinée à être apposée sur la peau S. La face de contact 10 est sensiblement planaire, au sens où elle s'étend parallèlement à un plan XY, certaines portions pouvant être inclinées par rapport au plan XY. Le corps principal comporte également une extrémité distale 4, opposée à la face de contact 10. La surface de contact 10 et l'extrémité distale 4 sont reliées l'une à l'autre par une face latérale 5, s'étendant autour d'un axe central $\Delta$, parallèle à un axe transversal Z, ce dernier étant perpendiculaire au plan XY.

La face de contact 10 peut être formée par une membrane perméable au $CO_2$ ou une plaque comportant des ouvertures d'admission 12 permettant un passage du $CO_2$. La face de contact peut comporter une membrane hydrophobe, de façon à éviter la diffusion de la vapeur d'eau à travers le dispositif 1.

Selon une possibilité, le dispositif comporte également un élément de chauffage, permettant de porter la face de contact 10, délimitant la chambre de mesure 20, à une température supérieure à 37°C, et de préférence comprise entre 40°C et 50°C, et de préférence entre 40°C et 45 °C, par exemple 42°C. L'élément de chauffage est par exemple une résistance ménagée sur la face de contact, produisant un chauffage par effet joule. Une augmentation locale et modérée de la température, au voisinage de la peau, favorise en effet une augmentation du débit sanguin par dilatation des capillaires sanguins, ce qui augmente la diffusion d'un gaz d'intérêt transcutané, à travers la peau.

La face de contact 10 débouche sur une chambre de mesure 20. La fonction de la chambre de mesure est d'estimer une concentration de $CO_2$ du mélange gazeux circulant dans le corps principal 2, parallèlement, ou sensiblement parallèlement, à l'axe central $\Delta$. A cette fin, la chambre de mesure 20 comporte un capteur de gaz 23. Plusieurs types de capteurs peuvent être utilisés à cette fin, par exemple des capteurs optiques ou des capteurs électrochimiques, ces derniers pouvant notamment être basés sur des oxydes métalliques (capteurs MOX), ou des capteurs photoacoustiques. Le capteur optique peut être un capteur fluorescent. Les inventeurs ont estimé qu'il était préférable d'utiliser un capteur optique, et plus précisément un capteur infra-rouge. Un tel capteur ne nécessite pas de maintenance particulière, et est particulièrement compact, ainsi que peu onéreux. De plus, un tel capteur est très spécifique pour caractériser des liaisons chimiques. Il convient à la détection de molécules de petites tailles, par exemple le dioxyde de carbone.

Le capteur de gaz est un capteur de type NDIR (Non Dispersive Infra Red). Ce type de capteur comporte une source de rayonnement infrarouge 24, émettant généralement dans une bande spectrale comprise entre 1 $\mu$m et 20 $\mu$m. Il comporte également au moins un photodétecteur de mesure 25, sensible au rayonnement infra-rouge. Le principe repose sur l'atténuation, par le gaz analysé, du rayonnement infra-rouge, émis par la source. La source infra-rouge 24 et le photodétecteur de mesure 25 forment le capteur de gaz 23. Le photodétecteur de mesure 25 est par exemple une thermopile. Un filtre 26 placé devant la thermopile détermine la longueur d'onde du rayonnement infrarouge mesuré par la thermopile. Différentes configurations du capteur de gaz sont décrites par la suite.

Le capteur de gaz 23 est configuré de telle façon que le gaz d'intérêt transcutané, en l'occurrence le $CO_2$, se propage entre la source de lumière et le photodétecteur, parallèlement à l'axe central $\Delta$, ou sensiblement parallèlement à l'axe central $\Delta$. Par sensiblement parallèlement, on entend parallèlement en admettant une tolérance angulaire inférieure à $\pm$ 30° ou à $\pm$ 20°.

La source infra-rouge émet une lumière se propageant perpendiculairement à l'axe transversal Z. De préférence, la lumière émise par la source est un faisceau en forme de nappe lumineuse perpendiculaire à l'axe transversal Z. Selon l'axe transversal Z, l'épaisseur du faisceau est de préférence inférieure à 1 cm, et de préférence inférieure à 5 mm. De préférence, la nappe lumineuse s'étend sur au moins 50 %, voire au moins 80% de la section transversale de la face de contact 10, cette dernière étant comprise entre quelques $cm^2$ et 25 ou 30 $cm^2$. Par section transversale, on entend une surface perpendiculaire à l'axe transversal Z (ou à l'axe central $\Delta$). La faible épaisseur du faisceau permet de diminuer le temps de réponse. La surface élevée de la section transversale du faisceau permet d'augmenter la quantité de gaz prélevée et donc la sensibilité de la mesure.

Afin de favoriser un écoulement selon l'axe transversal Z, à travers la chambre de mesure 20 la face de contact comporte une multitudes d'ouvertures d'admission 12, réparties selon une section transversale de quelques $cm^2$, par exemple entre 5 et 25 $cm^2$. Les ouvertures d'admission forment un facteur de transparence $r_{transpa\_mes}$. Le facteur de transparence correspond à la surface des ouvertures d'admission sur la surface de la face de contact. Cela correspond à une proportion de la face de contact ouverte sur le milieu à analyser. Alternativement, la face de contact supporte une membrane poreuse, perméable au $CO_2$. La chambre de mesure 20 débouche sur une chambre de collecte 30. L'interface entre la chambre de mesure 20 et la chambre de collecte 30 peut être formée par une ouverture de collecte large, ou par une pluralité d'ouvertures de collecte 22, ménagées dans une plaque de collecte 21 et réparties selon une section transversale de surface élevée, de façon similaire aux ouvertures d'admission 12. Chaque ouverture de collecte 22 débouche dans une chambre de collecte 30.

La chambre de collecte 30 comporte des ouvertures latérales 34, ménagées dans la face latérale 5 du dispositif 1. Les ouvertures latérales sont configurées pour permettre une admission d'air ambiant à l'intérieur du dispositif 1. L'air ambiant est l'air situé à l'extérieur du dispositif. Le flux d'air ambiant s'écoulant à travers les ouvertures latérales 34 est dirigé vers l'axe central $\Delta$. Chaque ouverture latérale 34 est de préférence orientée perpendiculairement à l'axe central $\Delta$, de telle façon que l'air ambiant pénètre dans le dispositif 1, à travers chaque ouverture latérale 34, selon une direction perpendiculaire, ou sensiblement perpendiculaire, à l'axe central Δ.

L'admission de l'air ambiant est obtenue par une pompe 41, agencée pour créer un transport du gaz carbonique par convection, dans la chambre de collecte, le gaz porteur pouvant être l'air ambiant ou éventuellement un autre gaz porteur comme de l'azote. L'air ambiant est introduit par les ouvertures latérales et entraîné vers une ouverture d'évacuation à travers la chambre de collecte. La pompe 41 peut être située dans un conduit d'évacuation 40, adjacent de la chambre de collecte 30. L'évacuation du gaz d'intérêt par le gaz porteur appauvrit le contenu en gaz d'intérêt de la chambre de collecte et diminue sa pression partielle. La différence de pression partielle du gaz d'intérêt entre la face de contact de la peau et la chambre de collecte induit l'écoulement par diffusion du gaz d'intérêt à travers la chambre de mesure 10 jusqu'à la chambre de collecte 20, à travers les ouvertures d'admission 12 et les ouvertures de collecte 22. De préférence, le débit du gaz porteur dans la chambre de collecte est ajusté de façon à ce que l'écoulement du gaz d'intérêt par diffusion à travers la chambre de mesure 20 soit laminaire. Le débit du gaz porteur dans la chambre de collecte peut valoir par exemple entre 0,1 et 10 mL/min, suivant le volume de la chambre.

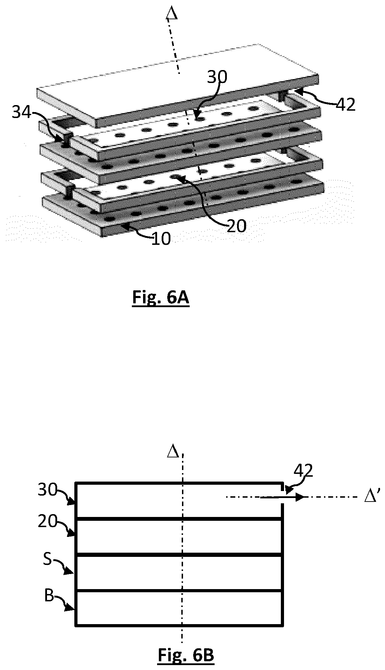

Dans la chambre de collecte 30, l'air ambiant, admis à travers les ouvertures latérales 34, se mélange avec le gaz d'intérêt, admis à travers les ouvertures de collecte 22. Par convection, l'air mélangé au gaz d'intérêt, est évacué dans le conduit d'évacuation, jusqu'à une ouverture d'évacuation 42, formant l'extrémité distale 4 du dispositif. Dans l'exemple représenté sur les figures 1A et 1B, l'air se propage à travers l'ouverture d'évacuation 42 autour de l'axe central Δ. Selon une autre possibilité, décrite en lien avec les figures 6A et 6B, l'air se propage à travers l'ouverture d'évacuation 42 selon un axe d'évacuation Δ' sécant, et notamment perpendiculaire, à l'axe central Δ.

Dans l'exemple représenté, les ouvertures latérales sont disposées sur deux faces opposées $5_1$, $5_2$ de la paroi latérale. Cela favorise une symétrie de l'écoulement d'air dans le dispositif. Toutefois, un tel agencement symétrique des ouvertures latérales n'est pas nécessaire. Les ouvertures latérales 34 peuvent n'être ménagées que sur une face de la paroi latérale, le conduit d'évacuation étant alors disposé sur une face opposée de la paroi latérale. Voir par exemple le mode de réalisation représenté sur la figure 6A.

Chaque ouverture latérale peut être associée à un filtre piégeant le gaz d'intérêt. Dans cet exemple, il s'agit d'un filtre à $CO_2$, par exemple un filtre comportant de la chaux, de façon à piéger le $CO_2$ présent dans l'air ambiant.

Le dispositif comporte une unité de traitement 50. L'unité de traitement 50 comporte des moyens de calcul, par exemple un microprocesseur ou un microcontrôleur, embarqué sur le dispositif, en étant relié par une liaison filaire ou sans fil à un téléphone portable ou à un calculateur de type PC. L'unité de traitement 50 est également configurée pour mettre en oeuvre le procédé d'estimation de la teneur en $CO_2$ dissous du sang de l'utilisateur, à partir de la concentration de $CO_2$ résultant du capteur de gaz 23.

Les principes généraux régissant l'estimation de la teneur en COz dissous dans le sang à partir de la concentration de $CO_2$ détectée dans le capteur de gaz ont été décrits dans WO2020/249466.

Le procédé comporte un calcul de la concentration du gaz carbonique dans la chambre de mesure à partir des mesures réalisées par les deux thermopiles à partir de modèles décrivant l'atténuation du rayonnement infrarouge aux deux longueurs d'onde associées à chacune des thermopiles (modèle de Beer Lambert, modèle linéaire quadratique). Ces modèles sont décrits dans WO2020/249466

Les mesures résultant du capteur peuvent être bruitées, auquel cas un filtrage passe-bas peut être appliqué. Le filtrage passe-bas peut être réalisé par exemple par un filtre à moyenne glissante dont la longueur est adaptée au niveau du bruit de mesure.

La teneur de CO2 dans le sang peut être estimée selon l'expression

$$C_{CO_2}^{in\,sang} = H^{sang} C_{CO_2}^{in\,col\,air}$$
$$+ \frac{Q^{air} + r_{transpa\_mes} A^{sang} K^{sang-peau-mes} H^{sang}}{r_{transpa\_mes} A^{sang} K^{sang-peau-mes}} \cdot \left[ C_{CO_2}^{out\,mes} - C_{CO_2}^{in\,col\,air} \right]$$

Où :

- $C_{CO_2}^{in\,sang}$ est la concentration du gaz carbonique dans le sang en entrée du compartiment sang ;
- $C_{CO_2}^{in\,col\,air}$ est la concentration du gaz carbonique dans l'air ambiant en entrée de la chambre de collecte. Cette concentration peut être mesurée par un capteur auxiliaire, destiné à déterminer la concentration de $CO_2$ dans l'air ambiant s'étendant autour du dispositif, comme décrit dans WO2020/249466. Selon une autre possibilité, un filtre

absorbant le $CO_2$ est disposé au niveau des ouvertures latérales 34. Dans ce cas, on suppose que $C_{CO_2}^{in\ col\ air} = 0$ ;

- $C_{CO_2}^{out\ mes}$ est la concentration du gaz carbonique dans la chambre de mesure ;
- $Q^{air}$ est le débit d'air dans la chambre de collecte ;
- $A^{sang}$ est la surface de contact entre le dispositif est la peau ;
- $K^{sang\text{-}peau\text{-}mes}$ est le coefficient de transfert de masse total à travers le sang, la peau et la chambre de mesure ;
- $H^{sang}$ est le coefficient de Henry adimensionnel définissant la solubilité du gaz carbonique dans le sang ;
- $r_{transpa\_mes}$ est le facteur de transparence de la grille entre la peau et la cellule de mesure ;

Nous en déduisons la pression $P_{CO_2}^{in\ sang}$ du CO2 dans le sang à partir de la concentration $C_{CO_2}^{in\ sang}$

$$P_{CO_2}^{in\ sang} = \frac{C_{CO_2}^{in\ sang}}{\beta^{sang}}$$

où :

- $\beta^{sang}$ est le coefficient de solubilité d'Ostwald du gaz carbonique dans le sang

La figure 1B schématise les courants fluidiques formés à l'intérieur du dispositif 1. Le $CO_2$ transcutané est admis dans le dispositif par les ouvertures d'admission 12 pratiquées dans la face de contact 10, et débouchant dans la chambre de mesure 20 (cf. flèche $F_1$). Le $CO_2$ se propage à travers la chambre de mesure par diffusion, parallèlement à l'axe central, ce dernier étant parallèle l'axe transversal Z. (cf. flèches $F_2$). L'air ambiant est admis à travers les ouvertures latérales 34 pratiquées dans la paroi latérale (cf. flèches $F_3$). Le mélange gazeux comportant l'air ambiant et le $CO_2$ transcutané est réalisé dans la chambre de collecte 30. Ce mélange se propage vers l'ouverture d'évacuation 42 (cf. flèches $F_4$) pour sortir du corps 2 du dispositif (cf. flèches $F_5$)

Un aspect important du dispositif est le recours à la pompe 41, qui permet de former un courant d'air au niveau de la chambre de collecte 30, pour évacuer le mélange air / gaz d'intérêt transcutané. Cela permet également d'accélérer la circulation d'air dans la chambre de collecte. Il en résulte un effet de pompage accélérant la diffusion du $CO_2$ du sang vers l'air, à travers la peau et la chambre de mesure. Il en résulte une diminution du temps de réponse du dispositif. Lorsque le milieu à analyser subit une augmentation en échelon de la concentration de $CO_2$, le temps de réponse du détecteur correspond à un temps de montée. Le temps de montée, correspond au temps pour réaliser entre 10% et 90% de la variation de la concentration du gaz carbonique dans la chambre de mesure après application de l'échelon. Il peut être estimé avec la formule approchée suivante:

$$\Delta t^{montée} = 2,197.\tau$$
$$= 2,197.\frac{1}{\frac{r_{transpa\_mes}A^{sang}}{V^{col} + V^{mes}}} \cdot \frac{1}{\frac{Q^{air}}{r_{transpa\_mes}A^{sang}} + K^{sang-peau-mes}H^{sang}}$$

où :

- $Q^{air}$ est le débit d'air dans la chambre de collecte ;
- $A^{sang}$ est la surface de contact entre le dispositif est la peau ;
- $K^{sang\text{-}peau\text{-}mes}$ est le coefficient de transfert de masse total à travers le sang, la peau et la chambre de mesure ;
- $H^{sang}$ est le coefficient de Henry adimensionnel définissant la solubilité du gaz carbonique dans le sang ;
- $V^{mes}$ est le volume de la chambre de mesure ;
- $V^{col}$ est le volume de la chambre de collecte ;
- $r_{transpa\_mes}$ est le facteur de transparence de la surface de contact entre la peau et la cellule de mesure. Le facteur de transparence correspond à la proportion de la surface de contact qui est ouverte : il s'agit du rapport entre la surface de la partie de la face de contact, ouverte sur le milieu à analyser (en l'occurrence la peau de l'utilisateur), laissant ainsi passer le gaz, sur la surface totale de la face de contact.

On considère que le corps principal présente une géométrie parallélépipédique, le rapport surface sur volume $\frac{A^{sang}}{V^{col}+V^{mes}}$ vaut :

$$\frac{A^{sang}}{V^{col} + V^{mes}} = \frac{1}{\Delta z^{col} + \Delta z^{mes}}$$

Où :

- $\Delta z^{col}$ est la hauteur de la chambre de collecte
- $\Delta z^{mes}$ est la hauteur de la chambre de mesure Plus ces hauteurs $\Delta z^{col}$ et $\Delta z^{mes}$ sont petites, plus le rapport surface sur volume $\frac{A^{sang}}{V^{col}+V^{mes}}$ est grand et plus le temps de montée est petit.

Cette expression montre aussi l'importance du facteur de transparence. Plus il est proche de 1, plus le temps de montée est court.

Comme précédemment indiqué, la hauteur selon laquelle s'étend la chambre de mesure, entre les ouvertures d'admission 12 et les ouvertures de collecte 22, est relativement faible, de préférence inférieure à 5 mm préférentiellement à 3 mm. Il en est de même de la chambre de collecte.

De façon plus générale, le dispositif présente un rapport surface sur volume élevé. Ainsi, la hauteur h, entre la face de contact 10 et la face supérieure de la chambre de collecte, est de préférence inférieure à 15 mm , préférentiellement à 8 mm

Un autre aspect important de l'invention est la minimisation de la distance entre la chambre de mesure 20 et la face de contact 10. Cela permet d'effectuer la mesure du $CO_2$ au plus près de la peau. Il s'agit d'une différence importante par rapport au dispositif décrit dans l'art antérieur, dans lequel une chambre de collecte, comportant des ouvertures latérales, s'étend entre la face de contact et la chambre de mesure. Le rapprochement de la chambre de mesure 20 par rapport à la peau permet d'améliorer le temps de réponse du dispositif.

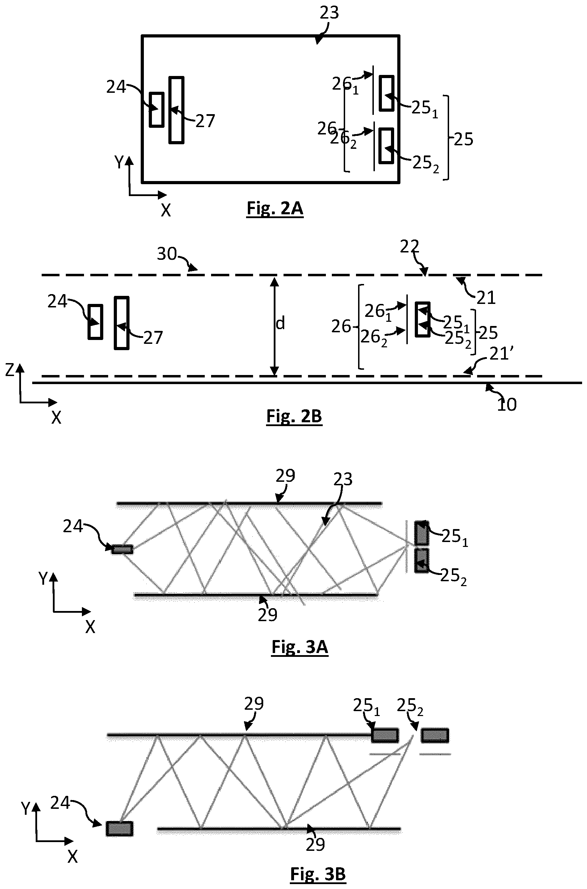

La figure 2A schématise la chambre de mesure 20, dans un plan $P_{XY}$ perpendiculaire à l'axe transversal. Elle définit un volume de mesure s'étendant entre une source de rayonnement infra-rouge 24 et le photodétecteur de mesure 25. La source et le détecteur sont des composants du capteur de $CO_2$ 23.

Le photodétecteur 25 comporte une voie de mesure $25_1$ et une voie de référence $25_2$. La voie de mesure $25_1$ est configurée pour détecter le rayonnement s'étant propagé à travers la chambre de mesure 20, dans une bande spectrale d'absorption qui correspond à l'espèce gazeuse que l'on souhaite analyser, en l'occurrence le $CO_2$. Ainsi, la voie de mesure $25_1$ effectue une mesure de l'intensité du rayonnement transmis par le mélange gazeux dans une bande spectrale de détection centrée sur $\lambda_1$ = 4.26 $\mu$m, définie par un filtre de mesure $26_1$. La voie de référence $25_2$ est configurée pour détecter le rayonnement s'étant propagé à travers la chambre de mesure 20 dans une bande spectrale de référence, dans laquelle l'absorption par le mélange gazeux est considérée comme négligeable. La bande spectrale de référence est par exemple centrée sur $\lambda_2$ = 3.91 $\mu$m . Elle est définie par un filtre de référence $26_2$. On note qu'à la longueur d'onde de détection $\lambda_1$ et à la longueur d'onde de référence $\lambda_2$, l'absorption du rayonnement par la vapeur d'eau peut être considérée comme égale. A défaut, la concentration d'humidité est prise en compte dans le modèle, comme décrit par la suite. Il en est de même de l'absorption de l'air, qui peut être considérée comme égale aux deux longueurs d'onde.

Afin d'élargir le faisceau, dans un plan perpendiculaire à l'axe transversal, la source infrarouge 24 peut être couplée à un diffuseur 27.

La chambre de mesure 20 comporte, de préférence, un capteur de température ainsi qu'un capteur d'humidité et un capteur de pression.

Le capteur de température sert à convertir la concentration mesurée en pression suivant la loi des gaz parfaits.

A l'aide de la loi des gaz parfaits, nous en déduisons la pression partielle du gaz carbonique $P_{CO_2}^{mes}(T^{mes})$ dans la chambre de mesure à la température $T^{mes}$ :

$$P_{CO_2}^{mes}(T^{mes}) = C_{CO_2}^{mes}.R.T^{mes}$$

Où $C_{CO_2}^{mes}$ est exprimée en (mol/m³), $R$ est la constante des gaz parfaits (0.0623637 m³.mmHg.K⁻¹.mol⁻¹), $T^{mes}$ en K et $P_{CO_2}^{mes}$ en mmHg.

Le capteur de pression atmosphérique $P_{air}^{mes}$ sert à calculer la pression relative $P_{CO_2}^{mes\ rel}$ exprimée en ppm (partie par million):

$$P_{CO_2}^{mes\ rel} = \frac{P_{CO_2}^{mes}}{P_{air}^{mes}} \cdot 10^6$$

Où $P_{CO_2}^{mes}$ et $P_{air}^{mes}$ sont exprimées dans la même unité, ici en mmHg.

Le capteur d'humidité sert à calculer la concentration de la vapeur d'eau dans la chambre de mesure. Un facteur d'atténuation du rayonnement important concerne la vapeur d'eau. La prise en compte de l'humidité nécessite de convertir la valeur d'humidité relative $RH^{mes}$ fournie par le capteur d'humidité en concentration de vapeur d'eau $C_{H_2O}^{mes}$ .L'humidité relative correspond à la pression partielle d'eau dans la chambre de mesure, notée $P_{H_2O}^{mes}(T^{mes})$ , sur la pression de vapeur saturante à la température $T^{mes}$ de la chambre de mesure.

$$RH^{mes} \triangleq \frac{P_{H_2O}^{mes}}{VP_{H2O}(T^{mes})} \cdot 100\%$$

La concentration en H₂O dans la chambre de mesure peut être obtenue selon l'expression :

$$P_{H_2O}^{mes} = C_{H_2O}^{mes} . R . T^{mes}$$

En combinant les deux équations précédentes, on obtient :

$$C_{H_2O}^{mes} = \frac{RH^{mes}.VP_{H2O}(T^{mes}).}{100\%.R.T^{mes}}$$

Les valeurs de pression de vapeur saturante $VP_{H2O}(T^{mes})$ en fonction de la température peuvent être obtenues à partir de tables, où à partir d'une expression analytique, par exemple l'équation de Tetens

$$VP_{H2O}(T^{mes}) = 0.61078 \, e^{\left(\frac{17.27\,(T^{mes}-273.15)}{T^{mes}-35.85}\right)}$$

La concentration $C_{H_2O}^{mes}$ peut être utilisée pour estimer la concentration de $CO_2$ dans la chambre de mesure, en utilisant l'expression de Beer-Lambert

$$-\ln\left[\frac{U(\lambda_1)/U(\lambda_2)}{U_0(\lambda_1)/U_0(\lambda_2)}\right]$$
$$= [k_{CO_2}(\lambda_1) - k_{CO_2}(\lambda_2)] . C_{CO_2}$$
$$+ [k_{H_2O}(\lambda_1) - k_{H_2O}(\lambda_2)] . C_{H_2O}$$
$$+ [A_{air}(\lambda_1) - A_{air}(\lambda_2)]$$

Où $C_{CO_2}$ et $C_{H_2O}$ sont les concentrations molaires, $k_{CO_2}(\lambda_1)$, $k_{CO_2}(\lambda_2)$, $k_{H_2O}(\lambda_1)$, $k_{H_2O}(\lambda_2)$ les coefficients d'atténuation du gaz carbonique et de la vapeur d'eau, $A_{air}(\lambda_1)$, $A_{air}(\lambda_2)$ les coefficients atténuations de l'air aux longueurs d'onde $\lambda_1$, $\lambda_2$.

Ainsi, la prise en compte de la concentration d'$H_2O$ permet d'améliorer la précision avec laquelle la concentration de $CO_2$ est mesurée, comme évoqué dans la demande de brevet WO2020/249466.

La figure 2B montre la chambre de mesure dans un plan Pxz parallèle à l'axe transversal Z. La chambre de mesure peut s'étendre entre deux grilles 21 et 21' perméables au gaz d'intérêt. La grille 21 forme la paroi d'interface avec la chambre de collecte 30. Elle comporte des ouvertures de collecte, permettant l'écoulement du gaz d'intérêt de la chambre de mesure vers la chambre de collecte. De préférence, chaque grille est réfléchissante dans la bande spectrale d'émission de la source infra-rouge. La chambre de mesure peut comporter une grille réfléchissante 21', similaire à la grille 21, au niveau de la face de contact 10. La présence de chaque grille réfléchissante permet d'augmenter la quantité de lumière se propageant à travers le gaz d'intérêt, vers chaque voie du photodétecteur. Elle permet également de limiter l'épaisseur de la nappe de lumière, selon l'axe transversal.

La distance d entre la grille 21 et la face de contact, ou entre les grilles 21 et 21', est par exemple comprise entre 3 mm et 8 mm.

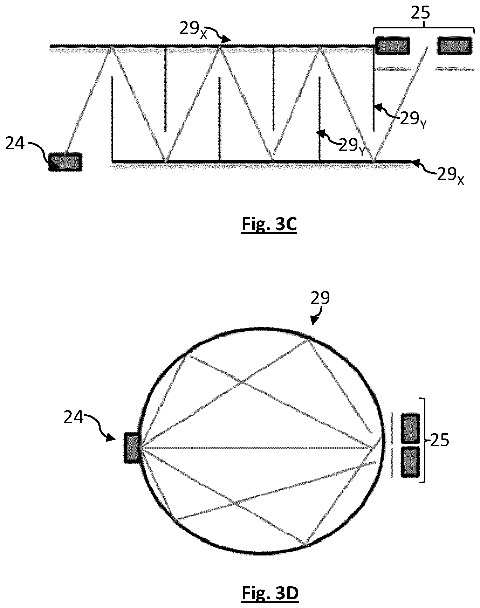

Les figures 3A à 3D illustrent différents agencements du capteur de gaz 23, permettant de former une nappe de lumière de faible épaisseur, s'étendant perpendiculairement à l'axe transversal Z. Selon l'axe latéral Y, la dimension de la nappe est limitée par des parois réfléchissantes 29. Sur les figures 3A et 3B, la source infra-rouge émet respectivement selon l'axe longitudinal X et l'axe latéral Y. Sur la figure 3C, la source infra-rouge émet à travers des parois réfléchissantes agencées en peignes entrelacés. Chaque paroi réfléchissante 29 présente un tronçon longitudinal $29_X$ s'étendant selon axe longitudinal X, à partir duquel une pluralité de cloisons transversales $29_Y$, parallèles à l'axe latéral Y, font saillie. Les parois sont disposées face à face, de telle sorte qu'une cloison transversale d'une paroi s'étend entre deux cloisons transversales adjacentes de la paroi opposée. Chaque cloison transversale n'atteint pas le tronçon longitudinal qui lui est opposée : un espace est laissé libre entre chaque cloison transversale et le tronçon longitudinal qui lui est opposé. L'espace permet un passage de la lumière entre l'extrémité de chaque cloison transversale et le tronçon longitudinal opposé. La lumière se propage successivement entre chaque cloison transversale et le tronçon longitudinal opposé Les cloisons transversales sont dites entrelacées ou imbriquées. Cette configuration permet d'allonger le chemin optique par rapport à un parcours direct suivant l'axe X, tout en assurant une moindre dispersion sur la longueur des chemins optiques des rayons émis par la source de lumière et détectés par chaque voie de mesure. La lumière émise par la source de lumière se propage ainsi entre les parois en suivant les canaux délimités par les portions transversales.

Sur la figure 3D, la nappe de lumière est délimitée par une paroi circulaire réfléchissante permettant d'assurer que tous les trajets entre la source et la thermopile situées de façon diamétralement opposée ont la même longueur.

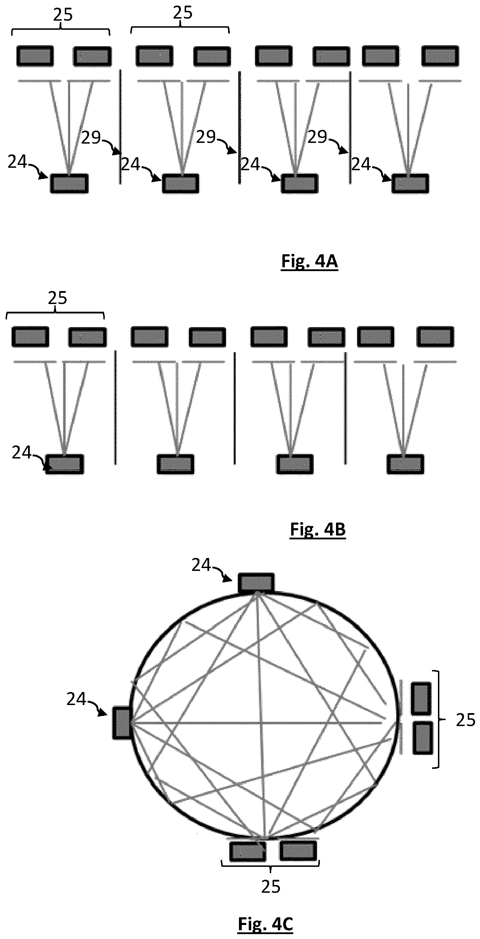

Les figures 4A à 4C représentent des configurations selon lesquelles on dispose de plusieurs ensembles pour paralléliser les mesures et améliorer la sensibilité du dispositif. Chaque ensemble comporte une source de lumière et un photodétecteur comportant deux voies de mesure, telles que précédemment décrites. Dans la configuration représentée sur la figure 4A, chaque ensemble est séparé l'un de l'autre par une paroi réfléchissante 29. Cela permet une moindre dispersion des chemins optiques des rayons émis par la source de lumière et détectés par chaque voie de mesure. Sur la figure 4B, on n'utilise pas de paroi réfléchissante de façon à augmenter la quantité de lumière reçue par chaque thermopile et d'augmenter la sensibilité. Ceci se fait au détriment de la non-linéarité introduite par la dispersion sur la longueur des chemins optiques suivant le positionnement de la source et de la thermopile. Sur la figure 4C, la nappe de lumière est délimitée par une paroi circulaire réfléchissante permettant d'assurer que tous les trajets entre la source et la thermopile situées de façon diamétralement opposée ont la même longueur. Elle comporte plusieurs ensembles source - thermopiles situés de manière diamétralement opposée pour paralléliser les mesures et améliorer la sensibilité.

La configuration optimale correspond à une géométrie où les longueurs du trajet entre la source est le détecteur sont les moins dispersées pour assurer une bonne linéarité des mesures, et ou la longueur du trajet est la plus longue pour assurer une bonne sensibilité. La configuration 3C semble optimale vis-à-vis de ces critères dans le cas où on utilise une seule source. Les parois servant de collimation limitent la dispersion et rallonge le trajet parcouru. Dans le cas où plusieurs sources peuvent être utilisées, la géométrie 4A est aussi celle qui minimise la dispersion sur les trajets parcourus du fait des collimateurs.

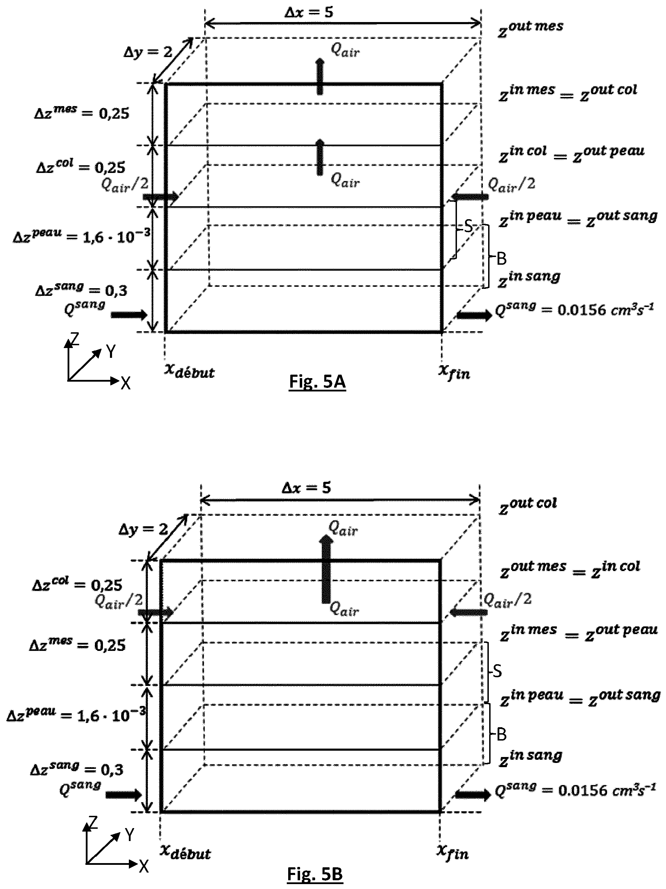

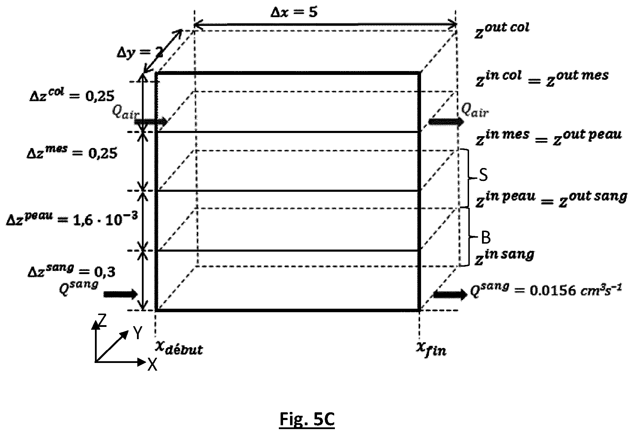

Les inventeurs ont simulé les performances de détection du détecteur tel que décrit dans WO2020/249466 et du détecteur selon l'invention. Les figures 5A, 5B et 5C schématisent respectivement :

- Le dispositif tel que décrit dans WO2020/249466 , représentatif de l'art antérieur ;
- Un premier mode de réalisation du dispositif selon l'invention « invention 1 » ;
- Un deuxième mode de réalisation du dispositif selon l'invention « invention 2 », le dispositif étant également représenté sur les figures 6A et 6B.

Quel que soit le dispositif, le sang occupe un compartiment sang B, entre les coordonnées $z^{in\ sang}$ et $z^{out\ sang}$, et son épaisseur est $\Delta z^{sang}$. Le compartiment sang B est recouvert par de la peau S. La peau S s'étend entre les coordonnées $z^{in\ peau}$ et $z^{out\ peau}$, et son épaisseur est $\Delta z^{peau}$. $z^{in\ peau} = z^{out\ sang}$. La peau est recouverte par le dispositif.

La chambre de collecte s'étend entre les coordonnées $z^{in\,col}$ et $z^{out\,col}$, et son épaisseur est $\Delta z^{col}$. La chambre de mesure s'étend entre les coordonnées $z^{in\,mes}$ et $z^{out\,mes}$, et son épaisseur est $\Delta z^{mes}$.

Dans le dispositif de l'art antérieur (figure 5A), le dispositif comporte une superposition de la chambre de collecte et de la chambre de mesure. $z^{in\,col} = z^{out\,col\,peau}$ et $z^{in\,mes} = z^{out\,col}$.

Selon l'invention (figures 5B et 5C), le dispositif comporte une superposition de la chambre de mesure 20 et de la chambre de collecte 30. Nous avons dans cette géométrie $z^{in\,mes} = z^{out\,peau}$ ; $z^{in\,col} = z^{out\,mes}$.

Les paramètres de dimensionnement du dispositif sont les suivants :

Spécification des paramètres :

- longueur (selon l'axe X) compartiment sang: $\Delta x^{sang} = \Delta x = 5$ cm ;
- largeur (selon l'axe Y) compartiment sang : $\Delta y^{sang} = \Delta y = 2$ cm ;
- hauteur compartiment sang (selon l'axe Z) : $\Delta z^{sang} = 0.3$ cm ;
- surface du compartiment sang au contact de la peau : $A^{sang} = 10$ cm$^2$ ;
- volume du compartiment sang : $V^{sang} = 3$ cm$^3$ ;
- Coefficient de Henry du gaz carbonique dans le sang : $H^{sang} = 0{,}54$
- Coefficient de diffusion du gaz carbonique dans le sang (coefficient de l'eau) : $D^{sang} = 2{,}2\ 10^{-5}$ cm$^2$.s$^{-1}$
- Concentration initiale en $CO_2$ dans le sang : $C^{in\,sang} = 1.0990$ $\mu$mol/cm3
- Débit sanguin : $Q^{sang} = 0{,}0156$ cm$^3$s$^{-1}$ qui correspond à la vitesse suivante:
- Vitesse transverse (selon X) du sang : $u_x^{sang} = 0{,}0259$ cm.s$^{-1}$
- longueur compartiment peau (selon l'axe X) : $\Delta x^{peau} = \Delta x = 5$ cm ;
- largeur compartiment peau (selon l'axe Y): $\Delta y^{peau} = \Delta y = 2$ cm ;
- hauteur compartiment peau (selon l'axe Z): $\Delta z^{peau} = 16\ 10^{-4}$ cm ;
- surface de la peau en contact avec le dispositif : $A^{peau} = 10$ cm$^2$ ;
- Coefficient de Henry du gaz carbonique dans la peau : $H^{peau} = 1{,}6$
- Coefficient de diffusion du gaz carbonique dans la peau : $D^{peau} = 1\ 10^{-7}$ cm$^2$.s$^{-1}$
- Température de la chambre de mesure : $T^{mes} = 315.15$ K ;
- Longueur de la chambre de mesure (selon l'axe X): $\Delta x^{mes} = \Delta x = 5$ cm ;
- Largeur de la chambre de mesure (selon l'axe Y): $\Delta y^{mes} = \Delta y = 2$ cm ;
- Hauteur de la chambre de mesure (selon l'axe Z): $\Delta z^{mes} = 0.25$ cm ;
- Coefficient de Henry du gaz carbonique dans la chambre de mesure : $H^{mes} = H^{air} = 1$
- Coefficient de diffusion du gaz carbonique dans la chambre de mesure : $D^{mes} = D^{air} = 0{,}18$ cm$^2$.s$^{-1}$
- Concentration en $CO_2$ dans l'air entrant, on suppose qu'il y a un filtre : $C^{air} = 0$
- Débit d'air convectif dans la chambre de mesure :

  - dans le cas de l'architecture de l'art antérieur : typiquement $Q^{air} = 1$ml/min : plage de variation de 0,1 à 5 ml/min. Ceci correspond à la vitesse axiale (selon Z) : typiquement $u_z^{mes} = 1{,}67.10^{-3}$ cm.s$^{-1}$
  - dans le cas de l'invention Débit d'air convectif dans la chambre de mesure: 0ml/min et vitesse axiale (selon z) : $u_z^{mes} = 0$ cm.s$^{-1}$

- Surface de la face de contact entre la chambre de mesure et la chambre de collecte : $A^{mes} = A^{col} = 10$ cm$^2$ ;
- Température de la chambre de collecte : $T^{col} = 315.15$ K ;
- Longueur de la chambre de collecte (selon l'axe X) : $\Delta x^{col} = \Delta x = 5$ cm ;
- Largeur de la chambre de collecte (selon l'axe Y): $\Delta y^{col} = \Delta y = 2$ cm ;
- Hauteur de la chambre de collecte (selon l'axe Z): $\Delta z^{col} = 0.25$ cm ;
- Coefficient de Henry du gaz carbonique dans la chambre de collecte : $H^{col} = H^{air} = 1$
- Coefficient de diffusion du gaz carbonique dans la chambre de collecte : $D^{col} = D^{air} = 0{,}18$ cm$^2$.s$^{-1}$
- Débit d'air convectif dans la chambre de collecte:

  ○ dans le cas de l'architecture de l'art antérieur : typiquement $Q^{air} = 1$ml/min : plage de variation de 0,1 à 5 ml/min. Ce qui correspond à la vitesse suivante :

    - Vitesse axiale (selon Z) : typiquement $u_z^{col} = 1{,}67.10^{-3}$ cm.s$^{-1}$

  ○ Débit d'air convectif dans la chambre de collecte dans le cas de l'invention : typiquement $Q^{air} = 1$ml/min : plage de variation de 0,1 à 5 ml/min. Ce qui correspond aux vitesses suivantes :

- Invention 1 : Vitesse axiale (selon Z) : typiquement $u_z^{col} = 1,67.10^{-3}$ cm.s$^-1$

- Invention 2 : Vitesse transverse (selon X) : $u_x^{col} = 0,033$ cm.s$^{-1}$

Le facteur de transparence à l'entrée de la chambre de mesure $r_{transpa\_mes}$ est égal à 0.3. Le facteur de transparence à l'entrée de la chambre de collecte $r_{transpa\_col}$ est également égal à 0.3.

L'objectif de la géométrie de la chambre de collecte est d'assurer une circulation d'air pour que l'air parcourt toute la surface d'échange entre la chambre de mesure et la chambre de collecte. Cette circulation d'air est réalisée entre des ouvertures d'admission d'air et des ouvertures d'évacuation d'air. Si la pompe est placée en amont de la chambre de collecte par rapport au circuit de circulation d'air, un tube ou plusieurs tubes assurent la connexion entre la pompe et la chambre de collecte à travers des ouvertures d'admission d'air. Si la pompe est placée en aval de la chambre de collecte par rapport au circuit de circulation d'air, un tube ou plusieurs tubes assurent la connexion entre la chambre de collecte et la pompe.

Dans le cas du transport avec évacuation axiale, le ou les trous d'évacuation sont placés dans le plafond de la chambre de collecte. Cette évacuation pourra être centrée si l'admission se fait sur au moins deux positions opposées pour que l'air parcourt toute la surface d'échange entre la chambre de mesure et la chambre de collecte. Elle pourra aussi être décentrée sur un côté opposé aux trous d'admission d'air pour que l'air puisse parcourir toute la surface d'échange entre la chambre de mesure et la chambre de collecte.

Dans le cas du transport avec évacuation transverse, le ou les trous d'évacuation sont situés sur une paroi latérale de manière opposée aux ouvertures d'admission pour que l'air parcourt toute la surface d'échange entre la chambre de mesure et la chambre de collecte.

Les trous d'admission et d'évacuation sont dimensionnés de façon à permettre de réaliser le débit d'air $Q^{air}$ recherché.

Dans le cas d'un dispositif avec évacuation axiale (figure 5A), la paroi supérieure de la chambre de collecte aura avantageusement une forme en entonnoir pour guider l'air vers le ou les trous d'évacuation.

Typiquement un trou d'admission ou d'évacuation d'air aura une surface comprise entre 1 et 10 $mm^2$. Ceci pourra représenter une surface totale d'admission ou d'évacuation pouvant aller jusqu'à 100 $mm^2$ si plusieurs trous en parallèle sont utilisés.

Nous présentons maintenant le modèle décrivant le transport du gaz carbonique du sang, puis à travers la peau, et à travers les trous d'évacuation de la chambre de collecte et la chambre de mesure. Ce modèle repose sur des équations de convection diffusion dans le sang et la chambre de collecte, et des équations de convection - diffusion dans la chambre de mesure pour la configuration de l'art antérieur, et des équations de diffusion dans la chambre de mesure pour les configurations invention 1 et invention 2.

Nous étudions un modèle dynamique, bidimensionnel suivant les coordonnées spatiales ($x$ selon l'axe X et $z$ selon l'axe Z), et dépendant du temps. Les variables de concentration dépendent des coordonnées spatiales $x$ et $z$, mais sont indépendantes de la coordonnée spatiale $y$. Dans ces équations, nous ne tiendrons compte que des coordonnées spatiales $x$ et $z$ et de la coordonnée temporelle $t$.

Equation de diffusion convection dans l'étape sang : valable pour les trois configurations (art antérieur, invention 1, invention 2)

$$\frac{\delta C_{CO_2}^{sang}}{\delta t} = -u_x^{sang} \frac{\delta C_{CO_2}^{sang}}{\delta x} + D^{sang} \left( \frac{\delta^2 C_{CO_2}^{sang}}{\delta x^2} + \frac{\delta^2 C_{CO_2}^{sang}}{\delta z^2} \right)$$

avec:

$$u_x^{sang} = \frac{Q^{sang}}{\Delta y^{sang} * \Delta z^{sang}}$$

Conditions de bord :

$$\frac{\delta C_{CO_2}^{sang}}{\delta x} \left( x = x^{début}, z^{in\ sang} \le z \le z^{out\ sang}, t \right) = 0,$$

$$\frac{\delta C_{CO_2}^{sang}}{\delta z}\left(x^{d\acute{e}but} \le x \le x^{fin}, z = z^{in\,sang}, t\right) = 0,$$

$$\frac{\delta C_{CO_2}^{sang}}{\delta x}\left(x = x^{fin}, z^{in\,sang} \le z \le z^{out\,sang}, t\right) = 0$$

$$C_{CO_2}^{sang}\left(x = x^{d\acute{e}but}, z^{in\,sang} \le z \le z^{out\,sang}, t\right) = C_{CO_2}^{in\,sang}(t)$$

Conditions à l'interface étage sang - étage peau :
Pour $x^{d\acute{e}but} \le x \le x^{fin}$

$$C_{CO_2}^{sang}(x, z = z^{out\,sang}, t) = \frac{H^{sang}}{H^{peau}} C_{CO_2}^{peau}\left(x, z = z^{in\,peau}, t\right)$$

$$D^{peau} \frac{\delta C_{CO_2}^{peau}}{\delta z}\left(x, z = z^{in\,peau}, t\right) = D^{sang} \frac{\delta C_{CO_2}^{sang}}{\delta z}\left(x, z = z^{out\,sang}, t\right)$$

<u>Equation de diffusion dans la peau</u> : valable pour les trois configurations (art antérieur, invention 1, invention 2)

$$\frac{\delta C_{CO_2}^{peau}}{\delta t} = D^{peau}\left(\frac{\delta^2 C_{CO_2}^{peau}}{\delta x^2} + \frac{\delta^2 C_{CO_2}^{peau}}{\delta z^2}\right)$$

Conditions de bord :

$$\frac{\delta C_{CO_2}^{peau}}{\delta x}\left(x = x^{d\acute{e}but}, z^{in\,peau} \le z \le z^{out\,peau}, t\right) = 0\,,$$

$$\frac{\delta C_{CO_2}^{peau}}{\delta x}\left(x = x^{fin}, z^{in\,peau} \le z \le z^{out\,peau}, t\right) = 0$$

<u>Equation de diffusion convection dans la chambre de collecte</u> :
Selon la configuration de l'art antérieur :

$$\frac{\delta C_{CO_2}^{col}}{\delta t} = -u_z^{col} \frac{\delta C_{CO_2}^{col}}{\delta z} + D^{air}\left(\frac{\delta^2 C_{CO_2}^{col}}{\delta x^2} + \frac{\delta^2 C_{CO_2}^{col}}{\delta z^2}\right)$$

avec $u_z^{col} = \frac{Q^{air}}{\Delta x^{col} * \Delta y^{col}}$

Conditions de bord

$$\frac{\delta C_{CO_2}^{col}}{\delta x}\left(x = x^{d\acute{e}but}, z^{in\,col} \le z \le z^{out\,col}, t\right) = 0\,,$$

$$\frac{\delta C_{CO_2}^{col}}{\delta x}\left(x = x^{fin}, \left(x = x^{d\acute{e}but}, z^{in\,col} \le z \le z^{out\,col}\right), t\right) = 0$$

$$C_{CO_2}^{col}\left(x^{début} \le x \le x^{fin}, z = z^{in\ col}, t\right) = C_{CO_2}^{in\ col\ air}(t)$$

On suppose que $C_{CO_2}^{in\ col\ air}(t)$ est une constante : la concentration de CO2 introduite à travers les parois latérales est constante.

Conditions à l'interface peau - chambre de collecte :

Pour $x^{début} \le x \le x^{fin}$

$$C_{CO_2}^{peau}\left(x, z = z^{out\ peau}, t\right) = H^{peau} C_{CO_2}^{col}\left(x, z = z^{in\ col}, t\right)$$

$$r_{transpacol} D^{peau} \frac{\delta C_{CO_2}^{peau}}{\delta z}\left(x, z = z^{out\ peau}, t\right) = D^{air} \frac{\delta C_{CO_2}^{col}}{\delta z}\left(x, z = z^{in\ col}, t\right)$$

- Invention 1 : la chambre de collecte est au-dessus de la chambre de mesure, la sortie d'air est axiale selon Z, les équations de transport sont les mêmes mais les conditions aux interfaces diffèrent.

$$\frac{\delta C_{CO_2}^{col}}{\delta t} = -u_z^{col} \frac{\delta C_{CO_2}^{col}}{\delta z} + D^{air}\left(\frac{\delta^2 C_{CO_2}^{col}}{\delta x^2} + \frac{\delta^2 C_{CO_2}^{col}}{\delta z^2}\right)$$

$$C_{CO_2}^{col}\left(x^{debut} \le x \le x^{fin}, z = z^{in\ col}, t\right) = C_{CO_2}^{in\ col\ air}(t)$$

avec $u_z^{col} = \dfrac{Q^{air}}{\Delta x^{col} * \Delta y^{col}}$

Conditions de bord

$$\frac{\delta C_{CO_2}^{col}}{\delta x}\left(x = x^{début}, z^{in\ col} \le z \le z^{out\ col}, t\right) = 0 ,$$

$$\frac{\delta C_{CO_2}^{col}}{\delta x}\left(x = x^{fin}, z^{in\ col} \le z \le z^{out\ col}, t\right) = 0,$$

$$\frac{\delta C_{CO_2}^{col}}{\delta z}\left(x^{début} \le x \le x^{fin}, z = z^{out\ col}, t\right) = 0$$

Conditions à l'interface chambre de mesure - chambre de collecte :

Pour $x^{début} \le x \le x^{fin}$

$$r_{transpa\_col} \frac{\delta C_{CO_2}^{mes}}{\delta z}\left(x, z = z^{out\ mes}, t\right) = \frac{\delta C_{CO_2}^{col}}{\delta z}\left(x, z = z^{in\ col}, t\right)$$

- Invention 2 : la chambre de collecte est au-dessus de la chambre de mesure, la sortie d'air est transverse selon X, les équations de transport changent par la direction du flux d'air, les conditions aux interfaces diffèrent.

$$\frac{\delta C_{CO_2}^{col}}{\delta t} = -u_x^{col} \frac{\delta C_{CO_2}^{col}}{\delta z} + D^{air}\left(\frac{\delta^2 C_{CO_2}^{col}}{\delta x^2} + \frac{\delta^2 C_{CO_2}^{col}}{\delta z^2}\right)$$

$$C^{col}\left(x = x^{debut}, z^{in\ col} \leq z < z^{out\ col}, t\right) = C_{CO_2}^{in\ col\ air}(t)$$

Avec

$$u_x^{col} = \frac{Q^{air}}{\Delta z^{col} * \Delta y^{col}}$$

Conditions de bord

$$\frac{\delta C_{CO_2}^{col}}{\delta x}\left(x = x^{début}, z^{in\ col} \leq z \leq z^{out\ col}, t\right) = 0\ ,$$

$$\frac{\delta C_{CO_2}^{col}}{\delta x}\left(x = x^{fin}, z^{in\ col} \leq z \leq z^{out\ col}, t\right) = 0,$$

$$\frac{\delta C_{CO_2}^{col}}{\delta z}\left(x^{début} \leq x \leq x^{fin}, z = z^{out\ col}, t\right) = 0$$

Conditions à l'interface chambre de mesure - chambre de collecte :
Pour $x^{début} \leq x \leq x^{fin}$

$$C_{CO_2}^{mes}(x, z = z^{out\ mes}, t) = C_{CO_2}^{col}\left(x, z = z^{in\ col}, t\right)$$

$$r_{transpa\_col} \frac{\delta C_{CO_2}^{mes}}{\delta z}(x, z = z^{out\ mes}, t) = \frac{\delta C_{CO_2}^{col}}{\delta z}\left(x, z = z^{in\ col}, t\right)$$

<u>Equation de diffusion convection dans la chambre de mesure</u>

- Art antérieur :

$$\frac{\delta C_{CO_2}^{mes}}{\delta t} = -u_z^{mes} \frac{\delta C_{CO_2}^{mes}}{\delta z} + D^{air}\left(\frac{\delta^2 C_{CO_2}^{mes}}{\delta x^2} + \frac{\delta^2 C_{CO_2}^{mes}}{\delta z^2}\right)$$

Avec

$$u_z^{mes} = \frac{Q^{air}}{\Delta x^{mes} * \Delta y^{mes}}$$

Conditions de bord

$$\frac{\delta C_{CO_2}^{mes}}{\delta x}\left(x = x^{début}, z^{in\ mes} \leq z \leq z^{out\ mes}, t\right) = 0\ ,$$

$$\frac{\delta C_{CO_2}^{mes}}{\delta x}\left(x = x^{fin}, z^{in\ mes} \leq z \leq z^{out\ mes}, t\right) = 0,$$

$$\frac{\delta C_{CO_2}^{mes}}{\delta z}\left(x^{d\acute{e}but} \leq x \leq x^{fin}, z = z^{out\,mes}, t\right) = 0$$

Conditions à l'interface chambre de collecte - chambre de mesure :
Pour $x^{d\acute{e}but} \leq x \leq x^{fin}$

$$C_{CO_2}^{col}\left(x, z = z^{out\,col}, t\right) = C_{CO_2}^{mes}\left(x, z = z^{in\,mes}, t\right)$$

$$r_{transpa\_mes}\frac{\delta C_{CO_2}^{col}}{\delta z}\left(x, z = z^{out\,col}, t\right) = \frac{\delta C_{CO_2}^{mes}}{\delta z}\left(x, z = z^{in\,mes}, t\right)$$

- Inventions 1 et 2 : la chambre de mesure est en contact avec la peau

$$\frac{\delta C_{CO_2}^{mes}}{\delta t} = D^{air}\left(\frac{\delta^2 C_{CO_2}^{mes}}{\delta x^2} + \frac{\delta^2 C_{CO_2}^{mes}}{\delta z^2}\right)$$

Conditions de bord

$$\frac{\delta C_{CO_2}^{mes}}{\delta x}\left(x = x^{d\acute{e}but}, z^{in\,mes} \leq z \leq z^{out\,mes}, t\right) = 0 \,,$$

$$\frac{\delta C_{CO_2}^{mes}}{\delta x}\left(x = x^{fin}, z^{in\,mes} \leq z \leq z^{out\,mes}, t\right) = 0$$

Conditions à l'interface peau - chambre de mesure :
Pour : $x^{d\acute{e}but} \leq x \leq x^{fin}$

$$C_{CO_2}^{peau}(x, z = z^{out\,peau}, t) = H^{peau} C_{CO_2}^{mes}\left(x, z = z^{in\,mes}, t\right)$$

$$r_{transpa\_mes}D^{peau}\frac{\delta C_{CO_2}^{peau}}{\delta z}(x, z = z^{out\,peau}, t) = D^{air}\frac{\delta C_{CO_2}^{mes}}{\delta z}\left(x, z = z^{in\,mes}, t\right)$$

Pour chaque configuration, on a procédé à des modélisations en utilisant un logiciel simulant le transport du gaz carbonique à partir du sang, à travers la peau vers les chambres de mesure et de collecte.
Dans l'architecture de l'art antérieur, le transport du COz gazeux est décrit par une équation de convection diffusion dans le compartiment sang, dans le compartiment chambre de collecte et dans le compartiment chambre de mesure et une équation de diffusion dans la peau. Dans l'architecture du dispositif selon l'invention, le transport du COz gazeux est décrit par une équation de diffusion dans la peau, et dans le compartiment chambre de mesure et une équation de convection diffusion dans le compartiment sang et dans le compartiment chambre de collecte.

Dans ce modèle bidimensionnel, le signal mesuré $\overline{C_{CO_2}^{mes}}(t)$ est calculé en sortie de la cellule de mesure en prenant la valeur moyenne de la concentration suivant la variable x :

$$\overline{C_{CO_2}^{mes}}(t) = \frac{1}{x^{fin} - x^{d\acute{e}but}}\int_{x^{d\acute{e}but}}^{x^{fin}} C_{CO_2}^{mes}(x, z = z^{out\,mes}, t)dx$$

On a estimé deux paramètres représentatifs des performances de chaque architecture :

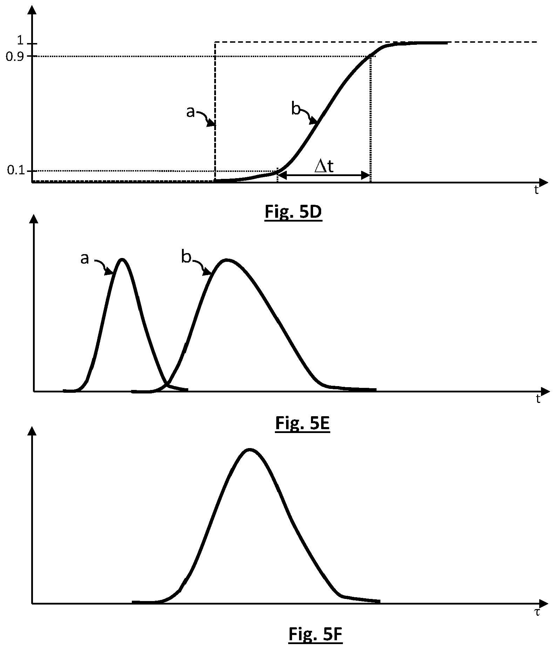

- le temps de montée Δt, qui correspond au temps nécessaire pour passer entre 10% et 90% de l'amplitude de variation de la concentration mesurée dans la chambre de mesure suite à une variation de pression partielle de COz formant un échelon entre une valeur basse et une valeur haute. Le temps de montée Δt est schématisé sur la figure 5D : la courbe a) schématise la réponse à l'échelon si le mesure était instantanée tandis que la courbe b) correspond à la réponse du capteur de gaz 23 en tenant compte de la cinétique du transport du gaz carbonique. Les réponses ont été normalisées sur la figure 5D pour que la valeur basse corresponde à 0 et la valeur haute à 1.
- le temps de retard τ qui correspond à un maximum de la fonction d'intercorrélation entre une impulsion de concentration dans le sang, représentée par exemple par une fonction Spline en forme de cloche comme représenté sur la courbe a de la figure 5E, et l'impulsion de la concentration mesurée par le capteur de gaz 23. Le temps de retard est assimilable à une durée de propagation du gaz carbonique entre le sang, la peau et la chambre de mesure. Sur la figure 5E, la courbe a) et la courbe b) schématisent respectivement l'impulsion de concentration dans le sang et l'impulsion de concentration mesurée par le capteur de gaz. Sur ce schéma les amplitudes des deux impulsions ont été normalisées à la même valeur. La figure 5F schématise une fonction d'intercorrélation entre les courbes a) et b) de la figure 5E. Le temps de retard τ retenu est celui correspondant au maximum de la fonction d'intercorrélation.

La fonction d'intercorrélation $\Gamma yz(\tau)$ entre un signal $y(t)$ et un signal $z(t)$ est de type :

$$\Gamma yz(\tau) = \frac{\lim_{T\to\infty}\frac{1}{2T}\int_{-T}^{T}\int \big(y(t)-\mu_y\big)\,(z(t-\tau)-\mu_z)dt}{\sigma_y\sigma_z}$$

où:

$$\mu_y = \lim_{T\to\infty}\frac{1}{2T}\int_{-T}^{T}y(t)dt$$

$$\mu_z = \lim_{T\to\infty}\frac{1}{2T}\int_{-T}^{T}z(t)dt$$

$$\sigma_y = \lim_{T\to\infty}\sqrt{\frac{1}{2T}\int_{-T}^{T}\big(y(t)-\mu_y\big)^2 dt}$$

$$\sigma_z = \lim_{T\to\infty}\sqrt{\frac{1}{2T}\int_{-T}^{T}(z(t)-\mu_z)^2 dt}$$

où $y(t)$ correspond à l'impulsion de concentration dans le sang et $z(t)$ correspond à l'impulsion de concentration mesurée par le capteur de gaz 23, dans la chambre de mesure.

On a modélisé l'évolution, dans le temps, de la concentration de $CO_2$ mesurée par la chambre de mesure de façon à déterminer le temps de montée et le temps de retard, ou retard global. Entre chaque modélisation, le débit d'air imposé par la pompe a été varié entre 1 mL/min et 5 mL/min. Le tableau 1 montre, pour chaque débit, le temps de montée et le retard global (ou temps de retard) simulés pour différentes valeurs de débit de la pompe 41.

Tableau 1

| Q_air (ml/min) | Paramètre étudié | Art Antérieur | Invention |
|---|---|---|---|
| 0,1 | Temps de montée (s) | 17152,8 | 7258,1 |
| | Retard global (s) | 445,1 | 376,5 |
| 0,2 | Temps de montée (s) | 9229,8 | 3769,4 |
| | Retard global (s) | 396,2 | 324,7 |

(suite)

| Q_air (ml/min) | Paramètre étudié | Art Antérieur | Invention |
|---|---|---|---|
| 0,5 | Temps de montée (s) | 3922,6 | 1606 |
| | Retard global (s) | 328,5 | 257,1 |
| 1 | Temps de montée (s) | 2051,8 | 879,3 |
| | Retard global (s) | 277,3 | 208,4 |
| 2 | Temps de montée (s) | 1100,9 | 528,8 |
| | Retard global (s) | 227,4 | 163,3 |
| 3 | Temps de montée (s) | 785,9 | 420,5 |
| | Retard global (s) | 199,6 | 139,3 |
| 4 | Temps de montée (s) | 631,9 | 369,5 |
| | Retard global (s) | 180,5 | 123,5 |
| 5 | Temps de montée (s) | 542,2 | 340,4 |
| | Retard global (s) | 166,3 | 112 |

On observe que les performances en termes de temps de montée et de retard global sont meilleures avec le dispositif selon l'invention qu'avec le dispositif dans la géométrie décrite dans l'art antérieur.

La tableau 2 montre les concentrations et pressions partielles de $CO_2$ mesurées par la chambre de mesure, alors que la concentration de $CO_2$ dans le sang correspond à une pression partielle de 40 mm de mercure.

Tableau 2

| Q_air (ml/min) | Paramètre étudié | Art antérieur | Invention 1 |
|---|---|---|---|
| 0,1 | Concentration ($\mu$mol/cm$^3$) | 0,26486 | 0,0874 |
| | Pression partielle (mmHg) | 5,2055 | 1,7185 |
| 0,2 | Concentration ($\mu$mol/cm$^3$) | 0,14164 | 0,0447 |
| | Pression partielle (mmHg) | 2,7839 | 0,8783 |
| 0,5 | Concentration ($\mu$mol/cm$^3$) | 0,05913 | 0,01813 |
| | Pression partielle (mmHg) | 1,1621 | 0,3563 |
| 1 | Concentration ($\mu$mol/cm$^3$) | 0,029999 | 0,0091146 |
| | Pression partielle (mmHg) | 0,5896 | 0,17914 |
| 2 | Concentration ($\mu$mol/cm$^3$) | 0,015111 | 0,004578 |
| | Pression partielle (mmHg) | 0,29699 | 0,089976 |
| 3 | Concentration ($\mu$mol/cm$^3$) | 0,010099 | 0,0030613 |
| | Pression partielle (mmHg) | 0,19848 | 0,060167 |
| 4 | Concentration ($\mu$mol/cm$^3$) | 0,0075836 | 0,0023021 |
| | Pression partielle (mmHg) | 0,14905 | 0,045246 |
| 5 | Concentration ($\mu$mol/cm$^3$) | 0,0060714 | 0,0018463 |
| | Pression partielle (mmHg) | 0,11933 | 0,036288 |

Le tableau 3 montre une comparaison des valeurs du temps de montée et du retard global en mettant en oeuvre les premier (invention 1) et deuxième mode de réalisation (invention 2). On observe que le deuxième mode de réalisation, selon lequel la sortie d'air est perpendiculaire à l'axe central, est plus favorable en terme de temps de montée et de retard global. Cependant, les concentrations et pressions partielles mesurées, pour une concentration dans le sang équivalente à une pression partielle de 40 mm de mercure, sont inférieures avec le deuxième mode de réalisation qu'avec le premier mode de réalisation (tableau 4).

Tableau 3

| Q_air (ml/min) | Paramètre étudié | Invention 1 | Invention 2 |
|---|---|---|---|
| 0,1 | Temps de montée (s) | 7258,1 | 4434,9 |
| | Retard global (s) | 376,5 | 336,8 |
| 0,2 | Temps de montée (s) | 3769,4 | 2288,1 |
| | Retard global (s) | 324,7 | 283,7 |
| 0,5 | Temps de montée (s) | 1606 | 980,7 |
| | Retard global (s) | 257,1 | 214,4 |
| 1 | Temps de montée (s) | 879,3 | 556,6 |
| | Retard global (s) | 208,4 | 164 |
| 2 | Temps de montée (s) | 528,8 | 363,7 |
| | Retard global (s) | 163,3 | 117,8 |
| 3 | Temps de montée (s) | 420,5 | 306,3 |
| | Retard global (s) | 139,3 | 94,2 |
| 4 | Temps de montée (s) | 369,5 | 280,1 |
| | Retard global (s) | 123,5 | 79,4 |
| 5 | Temps de montée (s) | 340,4 | 265,7 |
| | Retard global (s) | 112 | 69,4 |

Tableau 4

| Q_air (ml/min) | Paramètre étudié | Invention 1 | Invention 2 |
|---|---|---|---|
| 0,1 | Concentration ($\mu$mol/cm$^3$) | 0,0874 | 0,052896 |
| | Pression partielle (mmHg) | 1,7185 | 1,0396 |
| 0,2 | Concentration ($\mu$mol/cm$^3$) | 0,0447 | 0,02657 |
| | Pression partielle (mmHg) | 0,8783 | 0,52217 |
| 0,5 | Concentration ($\mu$mol/cm$^3$) | 0,01813 | 0,010463 |
| | Pression partielle (mmHg) | 0,3563 | 0,20563 |
| 1 | Concentration ($\mu$mol/cm$^3$) | 0,0091146 | 0,0050767 |
| | Pression partielle (mmHg) | 0,17914 | 0,099777 |
| 2 | Concentration ($\mu$mol/cm$^3$) | 0,004578 | 0,0024238 |
| | Pression partielle (mmHg) | 0,089976 | 0,047637 |
| 3 | Concentration ($\mu$mol/cm$^3$) | 0,0030613 | 0,0015655 |
| | Pression partielle (mmHg) | 0,060167 | 0,030769 |
| 4 | Concentration ($\mu$mol/cm$^3$) | 0,0023021 | 0,0011484 |
| | Pression partielle (mmHg) | 0,045246 | 0,02257 |

(suite)

| Q_air (ml/min) | Paramètre étudié | Invention 1 | Invention 2 |
|---|---|---|---|
| 5 | Concentration ($\mu$mol/cm$^3$) | 0,0018463 | 0,00090425 |
| | Pression partielle (mmHg) | 0,036288 | 0,017772 |

Bien que décrite en lien avec une mesure de $CO_2$ transcutané, l'invention peut être mise en oeuvre dans d'autres applications, de façon à mesurer un gaz émis par un milieu, solide ou liquide. Le milieu peut notamment être :

- un végétal, par exemple un fruit, de façon à suivre le procédé de maturation ;
- du lisier, de façon à suivre l'émission de gaz, par exemple le méthane ;
- un milieu de culture d'organismes ou de microorganismes biologiques ;
- de l'eau, par exemple de l'eau douce ou de l'eau de mer, par exemple pour suivre ou réguler la concentration de gaz;
- un sol, de façon à en étudier la respiration ;
- un milieu liquide destiné à l'élevage de poissons.

**Revendications**

1. Dispositif de mesure (1), destiné à être disposé contre un milieu, le dispositif s'étendant entre une face de contact (10), destinée à être appliquée en regard du milieu et une extrémité distale (4), le dispositif comportant une paroi latérale (5), s'étendant entre la face de contact et l'extrémité distale, le dispositif comportant :

   - au niveau de la face de contact (10), au moins une ouverture d'admission (12), configurée pour collecter un gaz d'intérêt émis à travers le milieu, l'ouverture d'admission étant pratiquée à travers la face de contact;
   - une chambre de mesure (20), comportant un capteur de gaz (23), le capteur de gaz étant configuré pour mesurer une concentration du gaz d'intérêt s'écoulant à travers la chambre de mesure;
   - une chambre de collecte (30), reliée à la chambre de mesure, et délimitée par une paroi latérale, la chambre de collecte comportant au moins une ouverture latérale (34), ménagée à travers la face latérale, ou sur une paroi supérieure de la chambre de collecte de façon à admettre un gaz dans la chambre de collecte ;

   le dispositif étant **caractérisé en ce que** :

   - la chambre de mesure (20) est disposée entre la face de contact (10) et la chambre de collecte (30) ;
   - le dispositif comporte un moyen d'entraînement (41), configuré pour entraîner le gaz porteur, admis à travers l'ouverture latérale, à travers la chambre de collecte, vers une ouverture d'évacuation (42), de façon que l'évacuation du gaz d'intérêt par le gaz porteur diminue la pression partielle du gaz d'intérêt dans la chambre de collecte, induisant un transport du gaz d'intérêt, par diffusion, de la face de contact vers la chambre de collecte, à travers la chambre de mesure.

2. Dispositif selon la revendication 1, comportant une source de chaleur, configurée pour porter la face de contact à une température supérieure à 37°C.

3. Dispositif selon la revendication 1 ou la revendication 2, dans lequel l'ouverture d'évacuation étant orientée autour d'un axe d'évacuation perpendiculaire ou perpendiculaire à 30° près, à la face de contact, la chambre de collecte débouchant sur l'ouverture d'évacuation.

4. Dispositif selon la revendication 1 ou la revendication 2, dans lequel l'ouverture d'évacuation est disposée à travers la paroi latérale de la chambre de collecte.

5. Dispositif selon l'une quelconque des revendications précédentes, dans lequel le capteur de gaz de la chambre de mesure est disposé à une distance inférieure à 8 mm de la face de contact.

6. Dispositif selon l'une quelconque des revendications précédentes, dans lequel le capteur de gaz (23) est un capteur optique, comportant une source infra-rouge (24), configurée pour émettre une lumière infra-rouge, et un photodétecteur (25), la chambre de mesure étant agencée de telle façon que le gaz d'intérêt s'écoule, dans la chambre de mesure (20), entre la source de rayonnement infra-rouge (24) et le photodétecteur (25), selon une direction d'écoulement perpendiculaire, ou perpendiculaire à ± 30° près, à la face de contact.

7. Dispositif selon la revendication 6, dans lequel la source de rayonnement infra-rouge est configurée pour produire une nappe de lumière parallèle, ou parallèle à ± 30° près à la face de contact.

8. Dispositif selon la revendication 7, dans lequel l'épaisseur de la nappe de lumière, selon un axe transversal perpendiculaire à la face de contact, est inférieure à 5 mm.

9. Dispositif selon la revendication 8, dans lequel la chambre de mesure comporte deux parois (21, 21'), parallèles à la face de contact, chaque paroi étant réfléchissante à l'égard de la lumière infra-rouge émise par la source infra-rouge, les parois délimitant une nappe de lumière dans laquelle se propage la lumière infra-rouge entre la source infra-rouge et le photodétecteur.

10. Dispositif selon la revendication 9, dans lequel une paroi réfléchissante forme la face de contact.

11. Dispositif selon la revendication 10 ou la revendication 9, dans lequel la paroi réfléchissante la plus proche de la face de contact est configurée pour être chauffée, de façon à chauffer le milieu.

12. Dispositif selon l'une quelconque des revendications 9 à 11, dans lequel :

• chaque paroi réfléchissante comporte des cloisons transversales ;
• chaque cloison transversale d'une paroi réfléchissante s'étend vers une paroi réfléchissante opposée,
• un espace est ménagé entre chaque cloison transversale et la paroi réfléchissante qui lui est opposée ;
• une cloison transversale d'une paroi réfléchissante s'étend entre deux cloisons transversales de la paroi réfléchissante opposée ;
• de façon que la lumière, émise par la source de lumière, se propage entre les cloisons transversales successives avant d'atteindre le détecteur.

13. Dispositif selon l'une quelconque des revendications précédentes, dans lequel le débit d'air de la pompe s'étend entre 0.1 et 10 mL/min.

14. Procédé d'estimation d'une teneur en gaz d'intérêt dans un milieu, à l'aide d'un dispositif (1) selon l'une quelconque des revendications précédentes, le dispositif étant appliqué de telle sorte que la face de contact (4) est disposée en regard du milieu, le moyen d'entraînement du gaz porteur étant activé, le procédé comportant:

a) une estimation d'une concentration de gaz d'intérêt dans la chambre de mesure (20);
b) à partir de la concentration du gaz d'intérêt dans la chambre de mesure (20), résultant de l'étape a), estimation d'une pression partielle du gaz d'intérêt dégagé le milieu.

15. Procédé selon la revendication 14, dans lequel le milieu est la peau d'un utilisateur, la peau s'étendant en dessus d'un vaisseau sanguin, dans lequel l'étape b) comporte :

(i) à partir concentration de gaz d'intérêt dans la chambre de mesure, résultant de l'étape a), estimation d'une concentration de gaz d'intérêt transcutané ;
(ii) à partir de la concentration de gaz d'intérêt transcutané résultant de la sous-étape (i), estimation d'une concentration ou pression partielle de gaz d'intérêt dissous dans le milieu.

**Fig. 1A**

**Fig. 1B**

**Fig. 2A**

**Fig. 2B**

**Fig. 3A**

**Fig. 3B**

**Fig. 3C**

**Fig. 3D**

**Fig. 4A**

**Fig. 4B**

**Fig. 4C**

$$\Delta x = 5$$

$$\Delta y = 2$$

$$\Delta z^{mes} = 0{,}25$$

$$\Delta z^{col} = 0{,}25$$
$$Q_{air}/2$$

$$\Delta z^{peau} = 1{,}6 \cdot 10^{-3}$$

$$\Delta z^{sang} = 0{,}3$$
$$Q^{sang}$$

$$Q_{air}$$
$$Q_{air}$$
$$Q_{air}/2$$

$$z^{out\,mes}$$
$$z^{in\,mes} = z^{out\,col}$$
$$z^{in\,col} = z^{out\,peau}$$
$$z^{in\,peau} = z^{out\,sang}$$
S
B
$$z^{in\,sang}$$
$$Q^{sang} = 0.0156\ cm^3 s^{-1}$$

$$x_{début}$$
$$x_{fin}$$

**Fig. 5A**

$$\Delta x = 5$$

$$\Delta y = 2$$

$$\Delta z^{col} = 0{,}25$$
$$Q_{air}/2$$

$$\Delta z^{mes} = 0{,}25$$

$$\Delta z^{peau} = 1{,}6 \cdot 10^{-3}$$

$$\Delta z^{sang} = 0{,}3$$
$$Q^{sang}$$

$$Q_{air}$$
$$Q_{air}$$
$$Q_{air}/2$$

$$z^{out\,col}$$
$$z^{out\,mes} = z^{in\,col}$$
$$z^{in\,mes} = z^{out\,peau}$$
$$z^{in\,peau} = z^{out\,sang}$$
S
B
$$z^{in\,sang}$$
$$Q^{sang} = 0.0156\ cm^3 s^{-1}$$

$$x_{début}$$
$$x_{fin}$$

**Fig. 5B**

**Fig. 5C**

**Fig. 5D**

**Fig. 5E**

**Fig. 5F**

Δ

30

42

34

20

10

**Fig. 6A**

Δ

42

30

Δ'

20

S

B

**Fig. 6B**

Europäisches
Patentamt
European
Patent Office
Office européen
des brevets

# RAPPORT DE RECHERCHE EUROPEENNE

Numéro de la demande

EP 23 19 3609

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2014/002823 A1 (NAKATANI SHIGERU [JP] ET AL) 2 janvier 2014 (2014-01-02) | 1,3,5-9, 13 | INV.<br>A61B5/145<br>G01N33/49 |
| Y | * alinéa [0031] – alinéa [0035] * | 2,4,10, 11 | |
| A | * alinéa [0043] – alinéa [0052] *<br>* figures 1,4 * | 12 | ADD.<br>A61B5/083 |
| | ----- | | A61B5/1455 |
| Y,D | WO 2020/249466 A1 (COMMISSARIAT ENERGIE ATOMIQUE [FR]) 17 décembre 2020 (2020-12-17) | 2,4,10, 11 | A61B5/1477<br>G01N33/00 |
| A | * page 2, ligne 10 – page 5, ligne 10 *<br>* page 7, ligne 20 – page 25, ligne 20 *<br>* figures 1A-6B * | 1,3,5-9, 12-15 | G01N33/02<br>G01N33/18<br>G01N33/24<br>G01N33/483 |
| | ----- | | |
| A | WO 2017/023500 A1 (VAIL SCIENT LLC [US]) 9 février 2017 (2017-02-09)<br>* alinéa [0023] *<br>* figure 3 * | 1-15 | |
| | ----- | | |
| A | JP 2010 148692 A (NAT CARDIOVASCULAR CT; TAIYO INSTR INC) 8 juillet 2010 (2010-07-08)<br>* alinéa [0015] – alinéa [0030] *<br>* figure 1 * | 1-15 | DOMAINES TECHNIQUES RECHERCHES (IPC) |
| | ----- | | A61B<br>G01N |
| A | WO 2014/005931 A1 (TOULOUSE INST NAT POLYTECH [FR] ET AL.) 9 janvier 2014 (2014-01-09)<br>* page 14, ligne 2 – ligne 18 * | 13 | G01V |
| | ----- | | |

Le présent rapport a été établi pour toutes les revendications

| Lieu de la recherche | Date d'achèvement de la recherche | Examinateur |
|---|---|---|
| La Haye | 11 janvier 2024 | Görlach, Tobias |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................................

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 23 19 3609

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-01-2024

| Document brevet cité au rapport de recherche | Date de publication | Membre(s) de la famille de brevet(s) | Date de publication |
|---|---|---|---|
| US 2014002823 A1 | 02-01-2014 | EP 2685230 A1 | 15-01-2014 |
| | | JP 5985465 B2 | 06-09-2016 |
| | | JP WO2012120957 A1 | 17-07-2014 |
| | | US 2014002823 A1 | 02-01-2014 |
| | | WO 2012120957 A1 | 13-09-2012 |
| WO 2020249466 A1 | 17-12-2020 | EP 3979911 A1 | 13-04-2022 |
| | | FR 3096885 A1 | 11-12-2020 |
| | | US 2022248992 A1 | 11-08-2022 |
| | | WO 2020249466 A1 | 17-12-2020 |
| WO 2017023500 A1 | 09-02-2017 | AUCUN | |
| JP 2010148692 A | 08-07-2010 | AUCUN | |
| WO 2014005931 A1 | 09-01-2014 | FR 2993051 A1 | 10-01-2014 |
| | | WO 2014005931 A1 | 09-01-2014 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2017023500 A
- JP 2010148692 B
- WO 2020249466 A